# EUROPEAN PATENT SPECIFICATION

(11) **EP 0 609 316 B1**
(45) Date of publication and mention of the grant of the patent: **16.04.1997**
(21) Application number: 92921768.5
(22) Date of filing: 26.10.1992
(51) Int. Cl.: G01N 33/00, G01N 27/12

(54) **SENSING GASEOUS SUBSTANCES**
NACHWEIS VON GASFÖRMIGEN SUBSTANZEN
DETECTION DE SUBSTANCES GAZEUSES

(30) Priority: 24.10.1991 GB 9122603
(43) Date of publication of application: 10.08.1994
(73) Proprietor: CAPTEUR SENSORS & ANALYSERS LTD., Didcot OX11 7HR (GB)
(72) Inventor: McGEEHIN, Peter School Road, Berkshire RG16 0QU (GB); WILLIAMS, David, Edward, Abingdon OX14 2EE (GB)
(74) Representative: Wendon, James
(86) International application number: GB9201963
(87) International publication number: WO9308467

(56) References cited:
- EP-A- 0 293 255
- WO-A-90/03569
- FR-A- 2 438 271

## Description

This invention relates to methods and apparatus for the sensing of gaseous substances in a gaseous mixture, for example the amounts of specific gases present in an exhaust from combustion apparatus.

In particular, the invention relates to the development of simplified arrays of two or more gas sensitive resistors, for utilisation in low-cost systems. Such systems might be utilised for example in the control of combustion apparatus, in particular the control of small boilers, to achieve maximum fuel economy and minimum pollutant emission.

Sensors in the form of gas-sensitive resistors, which utilise the fact that the electrical resistance of certain semiconducting oxides changes in response to change in composition of the gaseous atmosphere at elevated temperature, are cheap, robust and sensitive. They should therefore be admirably suited to deployment in combustion apparatus, such as boilers and particularly domestic and other such low-cost systems, for the purpose of monitoring the composition of the exhaust stream in order to control the supply of fuel to the burner with a view to maximising the combustion efficiency and minimising the emission of pollutant gases.

Such sensors as are known up to the present time are however generally not selective enough in their response to different gases to enable them to be so deployed satisfactorily. In particular, currently available sensors suffer from a strong response to water vapour in the gas stream. Since water vapour is a major and variable component of combustion gas exhaust, and indeed of the atmosphere generally, this sensitivity is a major drawback.

In order to overcome problems of lack of selectivity of these simple gas-sensitive resistors, it is known to use, for monitoring a plurality of gases in a mixture, arrays of sensing devices having somewhat different response characteristics from each other. By appropriate calibration and signal processing, for example by expressing the output of each sensor as a sum of responses due to each of the gases present in the mixture, and by solving the set of equations defined by the sensor responses and calibration coefficients, the concentration of the individual gases in the mixture may then be derived. Alternatively, pattern recognition methods might be employed.

By these means, it is conceivable that with a sufficiently complex apparatus and signal processing, the problem of monitoring and control of a complex gaseous mixture could be addressed. It is likely, however, that such an apparatus would be too complex and costly for installation in low-cost systems. The inherent advantage of cheapness of the basic gas sensitive resistance device would therefore have been lost.

WO-A-9 003 569 discloses an oxygensensor for automobile fumes. This device has two resistive elements arranged in series of parallel, the elements being of different types such that they exhibit opposite characteristics in response to a specific gas. FR-A-2 438 271 discloses a similar arrangement.

Some semiconducting oxides are n-type: that is, their normal response pattern is that their electrical resistance at the operation temperature decreases in the presence of reducing gases (such as carbon monoxide) in air, or with lowering of the partial pressure of oxygen, while the presence of oxidising gases such as nitrogen dioxide causes the resistance to increase. Well-known examples are SnO₂, WO₃₋ₓ and ZnO₂. Other examples may be found in the documents GB-A-2 149 120, GB-A-2 149 121, GB-A-2 149 122, GB-A-2 149 123, GB-A-2 166 244, and Chapter 4 of the book "Techniques and Mechanisms in Gas Sensing", Ed. P. T. Moseley, J. O. W. Norris and D. E. Williams (Adam Hilger:Bristol, 1991). Typical examples of these are the following: Cr_{1.9}Ti_{0.1}O₃₋ₓ; BaSnO₃; many compounds of the form Asn₁₋ₓBₓO_{3-z}, where A is an alkaline earth element and B is an element of valency 3 or 4, of which Gd and Zr are examples; compounds of the form A₂Sn₂O₇, where A is a rare earth element such as Y or Gd; complex, multicomponent oxides of niobium; and compounds such as the niobates and tantalates or iron, cobalt and nickel, e.g. FeNbO₄, CoNb₂O₆, NiNb₂O₆.

Other semiconducting oxides are p-type: that is, their normal response pattern is that their electrical resistance at the operating temperature increases in the presence of reducing gases (such as carbon monoxide) in air, or with lowering of the partial pressure of oxygen, while the presence of oxidising gases such as nitrogen dioxide causes the resistance to decrease. Examples include Ti_{0.9}Cr_{0.1}O_{1.95}, Bi₂Sn₂O₇. Other examples may be found in the documents cited above.

An object of this invention is to provide particularly simple ways of utilising gas sensitive resistor arrays. Simplification can be achieved by appropriate choice of the components of the array. The way in which this choice is made and the way in which these sensors are then connected together is a major feature of the invention.

According to the invention in a first aspect, an apparatus for sensing at least one gas in a mixture, comprises the features of claim 1.

The invention in a second aspect comprises a method of sensing at least one gas in a mixture according to claim 2. In both aspects of the invention, with appropriately chosen materials, the responses to interfering gases (particularly water vapour) cancel out.

The classification into p-type and n-type oxides is quite general and applies to many materials in their response to many different gases. However, certain materials do not show the normal response pattern of their type (n or p) in the presence of certain gases.

In this specification, such a response, in which the sign of the response curve (increasing, zero, or decreasing) is not that expected in the normal pattern, will be referred to as an "anomalous response", and a material giving such a response to at least one gas will be called an "anomalous material", though its response to other gases be normal. Examples of anomalous materials include BaTiO₃,
Basn₁₋ₓTiₓO₃(x>0.1), Basn₁₋ₓFeₓO₃(x>0.1), Ba₆FeNb₉O₃₀, and Bi₆Fe_{S}Nb₆O₃₀.

Some such materials, at the operating temperature, i.e. the temperature at which the response to traces of oxidising or reducing gases is optimal, are anomalous in that they show no significant response to a change in oxygen partial pressure over an important range of the latter, while their response to different oxidising or reducing gases is normal for their type. Thus, for example with an anomalous n-type material, some reducing gases cause the resistance to decrease, whereas others cause it to increase over the concentration range of interest, while the response to a reduction in oxygen partial pressure is either a reduction in the resistance (as is normal for an n-type material) or no discernable effect at the operating temperature at which the material is optimally sensitive to reducing gases. Examples of such materials include Ba₆Ti₂Nb₈O₃₀ and Sr₂NbO₄.

Similarly, with an anomalous p-type material, some reducing gases cause the resistance to increase, whereas others cause it to decrease over the concentration range of interest, while the response to a decrease in oxygen partial pressure is either an increase in resistance (as is normal for a p-type material), or no discernable effect at the operating temperature at which the material is optimally sensitive to reducing gases. One example of this kind of material is BaTiO₃.

Similar anomalous effects can be found with oxidising gases, some of which give resistance increases in certain p-type materials whilst others give resistance decreases. Similarly, some oxidising gases give resistance decreases in certain n-type materials, with others giving resistance increases. Examples include Ba₆FeNb₉F₃₀ and Bi₆FeNb₉O₃₀.

A normal n-type or p-type material in the context of a gas giving such an anomalous response in that material is included among those referred to herein as an anomalous material, since its response is then anomalous.

In some embodiments of the invention, at least one of the resistors has a response to the said gas that is uncharacteristic of its type of response (n or p) to at least one other gas.

It has been found that the anomalous effects discussed above can also be brought about by systematic substitution into the crystal lattice of a material having a normal p-type or n-type response. In addition, it is possible, by appropriate doping, to bring about a change in sign of the resistance response of a material to increasing gas concentration.

The invention is applicable to the detection and/or measurement, both of a single gas in a mixture and of more than one gas in a mixture, the resistor materials and connections being determined accordingly. In respect of a single gas, the use of more than one semiconductor resistor as sensors in an array has the effect of substantially increasing the range of response, as compared with a detecting or measuring device having only a single sensor.

In this connection a preferred feature of the invention is that the resistors are so chosen that their resistances are changed by substantially the same amount, and in the same sense, by the presence of at least one further gas in the mixture, so that the presence of such further gas or gases has a substantially zero effect on the output signal. There may be any number of these further gases, which are simply those that would interfere with the performance of the apparatus, or even prevent it from operating at all, for example because such interfering gas is present in very significant quantities. One example of such an interfering gas is water vapour, for example in the exhaust gases of a boiler or an internal combustion engine.

Many oxides, whether n- or p-type, show a decrease in their resistance in response to an increase in the partial pressure of water vapour in the gas mixture. Examples here include SnO₂, BaSnO₃ and FeNbO₄ (n-type) and Y₂TiFeO₇ (p-type).

Two ways in which these properties can be used in simple gas sensor arrays according to the invention adapted to particular tasks are suggested below. Such tasks include, for example, that of monitoring the exhaust gas of a boiler in order to provide a feedback signal for control of the burner, so as to attain the optimum combination of fuel efficiency and pollutant emission. With appropriate materials, it is possible to mount the sensors comprising the array onto a common substrate carrying a heater, such that the devices then operate at the same temperature. Such an arrangement also bestows advantages of cost and size.

In the first of these two ways, two sensors, one comprising a p-type material and the other an n-type material, are placed in the exhaust stream, and the difference in resistance of the two is measured. Since variations in the partial pressure of water vapour cause resistance decreases in both sensors, with the sensor geometry and resistance suitably adjusted the effects of water vapour variation can be made to cancel in the difference output. On the other hand, since the effects of the desired analyte gas or gases is to cause the resistances to change in opposite senses in the two sensors, the signal will be magnified in the difference output. A simple combination of appropriately chosen materials can thus be made in such a manner that interference from water vapour is minimised relative to the signal from the gaseous component of interest. One example of such a combination is SnO₂ and Y₂TiFeO₇. Another example is FeNbO₄ and Y₂TiFeO₇.

In the second, and more general, approach, two sensors are chosen from materials such that one has a normal response and the other an anomalous response, and such that the signals from the desired gaseous component comprise resistance changes of opposite signs on the two sensors, whilst the signals from the significant interfering components of the mixture comprise resistance changes of the same sign for the two sensors. The difference signal then enhances the response of the desired gas above those of the interfering gases. For example, a combination of a normal n-type material such as BaSnO₃, or FeNbO₄, or SnO₂, with an anomalous one such as Ba₆FeNb₉O₃₀ or Bi₆FeNb₉O₃₀ would serve to cancel the signals due to carbon monoxide and hydrocarbons, and enhance those due to oxides of nitrogen. Other combinations can readily be deduced which enhance the response to hydrocarbons whilst diminishing that due to carbon monoxide, and vice versa.

An extension of this principle is a combination in which the response to the target gas (the gas to be detected and/or measured) is anomalously small on one of the sensors but not on the other, whilst the responses to the principal interfering gases are of similar size and of the same sign on the two sensors. Such effects can be achieved by doping one of the sensors with suitable catalytic additives, such as Pt or Pd, or combinations of these. In such a case, the difference signal between the two sensors will again substantially cancel out any effects due to interfering gases, leaving only the signal due to the target gas. The desired effects might also be achieved by operating the two sensors at different temperatures, either by inducing a temperature gradient in a common substrate upon which the devices are mounted, or by having two separate devices.

Characteristics of the measurement problem itself may be combined with particular characteristics of the gas sensors in a simple measurement system according to the invention which addresses a particular control task. Typical of such an application is one in which it is required to detect the proportions of an oxidising gas and a reducing gas in the gas mixture.

Thus in some forms of the apparatus of the invention, one resistor has the same type (n or p) of response to the oxidising gas as the other resistor has to the reducing gas, so that the output signal is defined on a monotonic characteristic curve.

Alternatively, it can be arranged that one resistor has an n-type or p-type response to the oxidising gas, and the other has a response of a different type to the reducing gas, so that the output signal is defined on a characteristic curve having a maximum or minimum.

For example, in the control of a boiler, as the excess of air over fuel is increased, the combustion efficiency first rises and then falls, so that there is an optimum air/fuel ratio for optimum efficiency. As the air/fuel ratio increases, the concentration of oxygen in the exhaust stream increases and that of carbon monoxide and unburnt hydrocarbons decreases, whilst that of oxides of nitrogen goes through a maximum. The aim therefore is to achieve optimum thermal efficiency compatible with minimal pollutant emission. It has been found that the best indicators of this condition for use in the control of the boiler are the concentrations of oxygen and of carbon monoxide in the gas stream.

Various embodiments of the invention will now be described, by way of example only and with reference to the accompanying drawings, in which:
Figure 1 shows variations in oxygen and carbon monoxide concentration in an exhaust gas, plotted against air/fuel ratio;
Figure 2 shows typical response curves for various gas-sensitive semiconductor resistors, with their resistance plotted against air/fuel ratio;
Figure 3 shows curves of typical output signals from an array of two sensors according to the invention in various forms, with the magnitude of the resistance output signal plotted against air/fuel ratio;
Figures 4 and 5 show diagrammatically an apparatus according to the invention in two forms; and
Figures 6 and 7 illustrate diagrammatically two modifications.

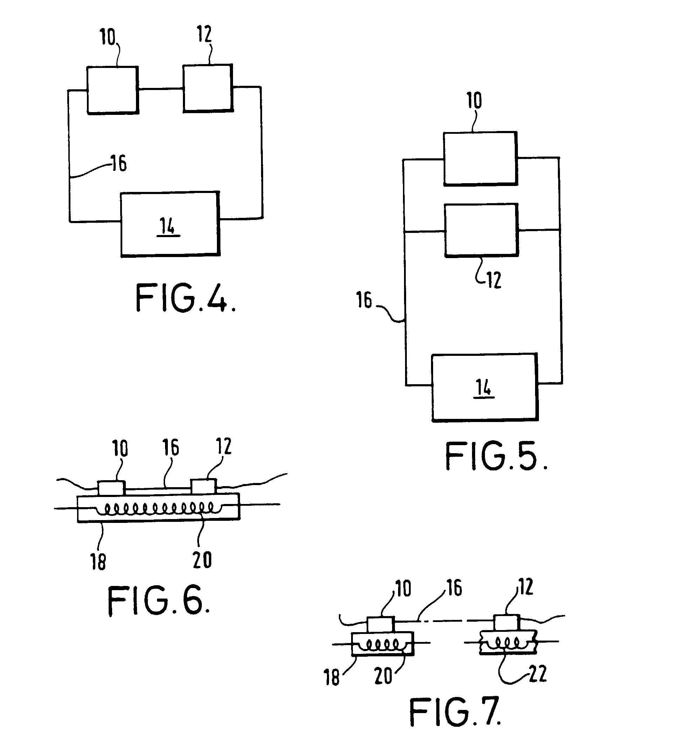

The arrangement now to be described provides for a choice of sensor elements, and a connection mode which provides a single control signal, adjustable so that the boiler will run so as to produce a certain ratio of oxygen to carbon monoxide in the exhaust gases. In this arrangement, the materials of the two sensor elements (and their operating temperature) are chosen so that one of them provides a response to oxygen and not carbon monoxide, while the other has a response to carbon monoxide but not to oxygen. These two elements 10, 12 are then connected either in series (Figure 4) or parallel (Figure 5), and the resistance of the resulting combination provides the desired control signal via the network connections 16 to the control system 14, which is adjusted so as to maintain this resultant resistance at a constant value.

Then, since the concentrations of oxygen and carbon monoxide change in the opposite sense with alteration of the air/fuel ratio, the maintenance of this constant value of the control resistance will achieve the desired objective of keeping the concentration of each of these gases in the exhaust (not shown) at its optimum value.

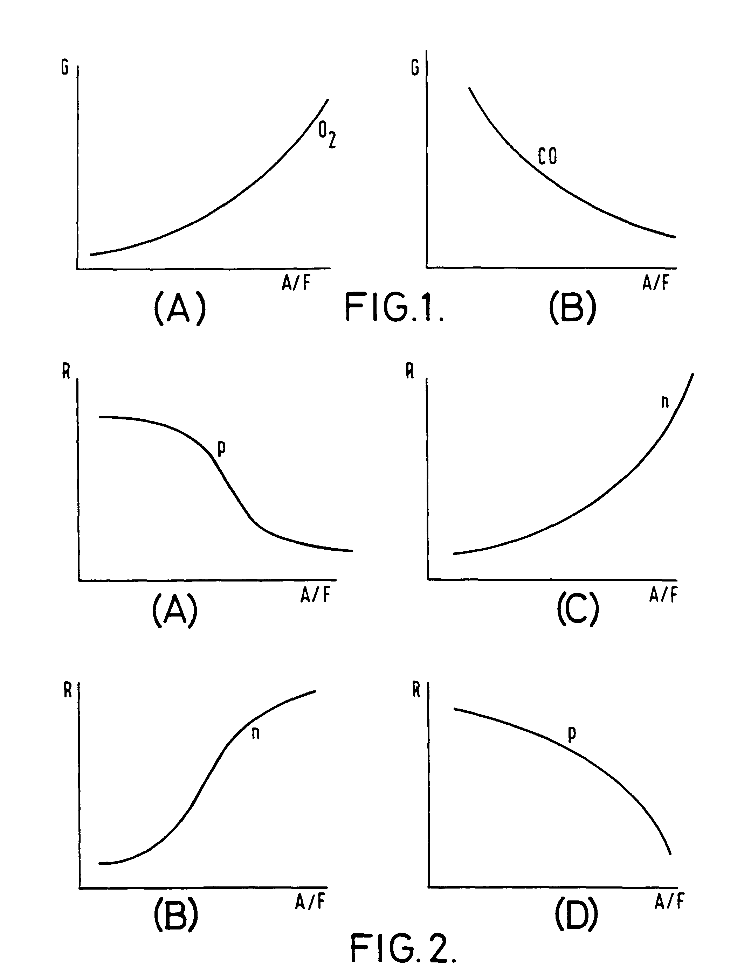

The various curves shown in Figures 1 to 3 show, by way of example, how the transfer functions of particular combinations of two sensor elements, having contrasting properties and being connected in series or in parallel can be determined in the case of a combustion monitoring application.

In Figure 1(A) the variation in oxygen concentration G in the exhaust as a function of input air/fuel ratio (A/F) is shown schematically (oxygen concentration rises with air/fuel ratio); for carbon monoxide the opposite is the case, as is seen in Figure 1(B).

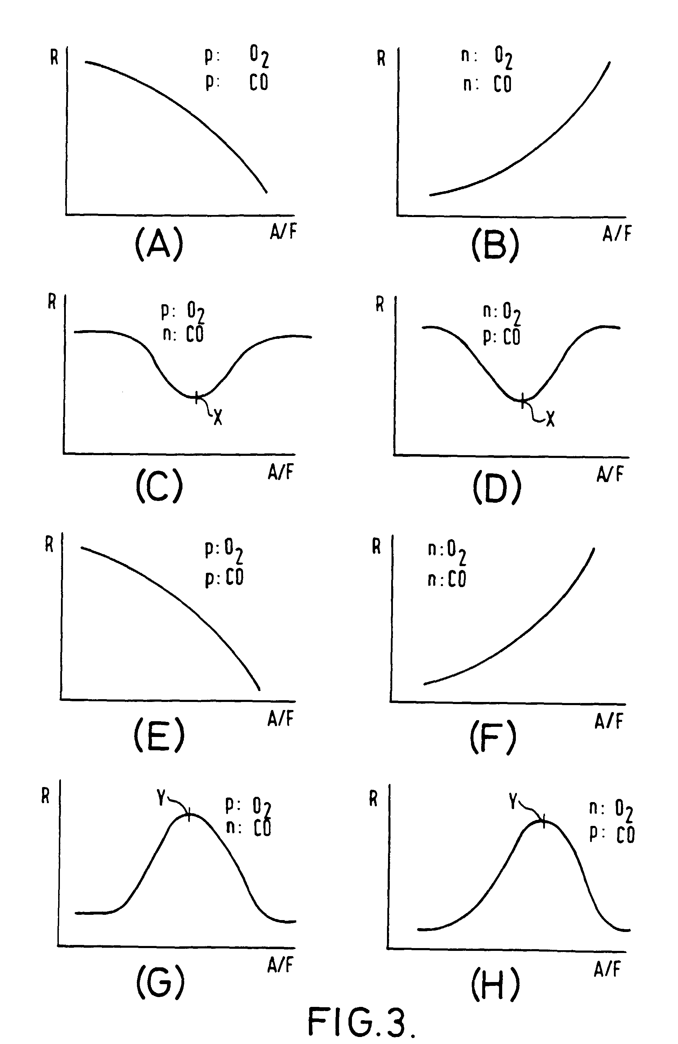

In Figure 2 the responses of sensors of particular types are sketched, the resistance R of each sensor, when placed in the exhaust, being plotted against the input air/fuel ratio (A/F). Figures 2(A) and 2(B) refer to higher temperature 'equilibrium' sensor devices with, respectively, p-type and n-type semiconductor responses, primarily to oxygen. Examples of materials for Figure 2(A) are X(Y_{1-z}Fe₂)O₃₋ₓ, where X is Ba or Sr, and Y is Ta or Ti, and Z is in the range 0 to 0.45 (for Ta) or 0 to 0.7 (for Ti). An example of a material for Figure 2(B) is TiO₂. For lower temperature 'non-equilibrium' devices responding primarily to carbon monoxide, the responses for materials classified as n-type and p-type are shown in Figures 2(C) and 2(D) respectively. Examples of materials for Figures 2(C) and 2(D) are SnO₂ and Ti_{0.9}Cr_{0.1}O_{1.95} respectively. The way in which resistance tends to vary with air/fuel ratio can for example be seen to be similar in Figures 2(A) and 2(D), and again in Figures 2(B) and 2(C), with decrease and increase, respectively, in resistance with increasing air/fuel ratio.

Figure 3 shows schematically the transfer functions which will be obtained by combining, either in series or in parallel, two sensors with characteristics chosen from Figure 2. Figures 3(A) to 3(D) show the case in which the sensors are in series, while in Figures 3(E) to 3(H) they are in parallel. In each case, one of the sensors (I) is responsive primarily to oxygen, while the other sensor (II) is responsive primarily to carbon monoxide. It should however be noted that each sensor may be such that it does respond to the other gas to some extent. The responses are as follows for the two sensors.

| Figures | Response type for the gases indicated | |
|---|---|---|
| | Sensor I (O₂) | Sensor II (CO) |
| 3(A) and 3(E) | p | p |
| 3(B) and 3(F) | n | n |
| 3(C) and 3(G) | p | n |
| 3(D) and 3(H) | n | p |

These sensor arrays provide a simple variation in resistance, as measured at the two output terminals of the array, which are more useful in practice than with single sensors. Thus, in the case illustrated in Figures 3(A) and 3(E), and in that of Figures 3(B) and 3(F), the curve of output resistance against air/fuel ratio is advantageously monotonic over a very wide field of air/fuel ratios. In Figures 3(C), 3(G), 3(D) and 3(H), however, there is a change in sign of the gradient of the curve representing resistance as a function of air/fuel ratio. This allows control to be accomplished near to the minimum and maximum points, indicated at X in Figures 3(C) and (D) and at Y in Figures 3(G) and (H) respectively, at which the sign of the gradient changes, by means of either a set-resistance or a differentiation method, both known per se, which may for example be used to effect corrective modulation of the input air/fuel ratio.

It will be apparent that, more generally, the use of curves such as those in Figure 3 and the use of both normal and anomalous properties of gas-sensitive oxides, with parallel or series connection (whichever is the more convenient, or is found to give the best results, in particular circumstances) of the sensors in the array, allows great flexibility to be obtained in the design of very simple arrays adapted for particular control and monitoring tasks.

Figure 6 shows two sensors 10, 12 mounted on a common substrate 18 containing a heating element 20 for maintaining both sensors at the same temperature, as discussed earlier herein. In Figure 6, the heating element may be graded so as to produce a difference in the temperatures at the two elements. Figure 7 shows the sensor 10 mounted on a heated substrate, the other sensor 12 being independently heated by a heating element 22, so that there is a difference in temperature between them, again as mentioned earlier.

Examples 1 to 5 below describe arrays in which the difference in output of two sensors is employed to enhance the signal due to the gas constituent to be measured, and to depress that for interfering gas constituents. The important characteristic is that for one of the elements the sign of the resistance response to the gas constituent to be measured is opposite to that for the non-relevant ("interfering") constituent of the mixture, whereas for the other element the responses are of the same sign.

In Examples 1 to 5, the sensor elements are chosen so as to cancel interference due to water vapour in the detection of hydrocarbons and carbon monoxide by operating them at a variable temperature. The sensor elements are chosen to enhance the response of the array to carbon monoxide and to diminish its response to the interfering signal from methane, by operating them at temperatures different from that required to cancel out water vapour effects.

In Example 6, the sensor elements are chosen so as to cancel the effects of water vapour, hydrocarbon and carbon monoxide emission, and to enhance the effects due to oxides of nitrogen.

The examples given in Figures 2 and 3 describe arrays in which different connection modes of the sensors are used to provide control signals suitable for boiler combustion control.

### Example 1

The sensor elements are of SnO₂ and Y₂TiFeO₇ respectively.

### Example 2

The sensor elements are of SnO₂ and Bi₂Sn₂O₇ respectively.

### Example 3

The sensor elements are of BaSnO₃ and Bi₂Sn₂O₇ respectively.

### Example 4

The sensor elements are of FeNbO₄ and Y₂TiFeO₇ respectively.

### Example 5

The sensor elements are of FeNbO₄ and Bi₂Sn₂O₇ respectively.

In all of Examples 1 to 5, signals due to water vapour are cancelled where the sensor array is responding to reducing gases such as CO and methane (depending on operating temperature).

### Example 6

One sensor element is of BaSnO₃ or of FeNbO₄ or SnO₂, the other being of Ba₆FeNb₉O₃₀ or Bi₆FeNb₉O₃₀. Signals due to gases other than NO₂ are cancelled.

In all cases, the operating temperature is generally in the range 300-500°C.

It will be appreciated from the foregoing that an array according to the invention need not have only two sensors, but that for more complex applications where several gases are to be monitored, any number of sensors, chosen for their responses to the gases in the mixture, may be connected in series or in parallel, or variously in series and in parallel. There may for example be two pairs of sensors, one pair for monitoring one or more particular gases, and the other for a further constituent of the mixture that is present in a much lower concentration, the sensors of each pair being so chosen as to be insensitive to, or to cancel out the effects of, the constituent or constituents of the mixture to be detected by the other pair.

## Claims

1. Apparatus for sensing at least one gas in a mixture, comprising an array of at least two gas-sensitive semiconductor resistors connected for giving an electrical output signal from the array in response to the concentration of at least one said gas, wherein a first said resistor has a response, selective or otherwise, of a first type, p or n, to a said gas, and a second said resistor has a response, selective or otherwise, of another type, n or p, to the same gas, the resistors being so connected that the output signal represents the difference between their instantaneous resistances, characterised in that the materials of the said first and second resistors are selected from the following combinations:
(i) the first and second resistors are of SnO₂ and Y₂TiFeO₇ respectively;
(ii) the first and second resistors are of SnO₂ and Bi₂Sn₂O₇ respectively;
(iii) the first and second resistors are of BaSnO₃ and Bi₂Sn₂O₇ respectively;
(iv) the first and second resistors are of FeNbO₄ and Y₂TiFeO₇ respectively;
(v) the first and second resistors are of FeNbO₄ and BiSn₂O₇ respectively;
(vi) the apparatus being suitable for the selective sensing of NO₂, the first resistor is of a material selected from BaSnO₃, FeNbO₄ and SnO₂, and the second resistor is of a material selected from Ba₆FeNb₉O₃₀ and Bi₆FeNb₉O₃₀.

2. A method of sensing at least one gas in a mixture by means of an array of gas-sensitive semiconductor resistors, characterised in that the array is an array according to Claim 1, the method comprising the steps of introducing the mixture simultaneously to the first resistor to produce a response of the said first type, and to the second resistor to produce a response of the said other type, and measuring the difference between the resulting resistance signals from the resistors.

3. A method according to Claim 2, in which the materials of the first and second sensors are selected as defined at (vi) in Claim 1, characterised in that the difference between the resulting resistance signals is used as a measure of the presence of NO₂ in the mixture.

4. A method according to Claim 2 or Claim 3, characterised in that the difference between the said signals is measured while the resistors are at different temperatures.

## Patentansprüche

1. Vorrichtung zum Erfassen wenigstens eines Gases in einer Mischung, mit einer Anordnung von wenigstens zwei gaserfassenden Halbleiterwiderständen, die zum Abgeben eines elektrischen Ausgangssignales aus der Anordnung in Reaktion auf die Konzentration wenigstens eines der Gase verbunden sind, wobei ein erster Widerstand eine Reaktion, selektiv oder in anderer Weise, einer ersten Art, p oder n, auf ein Gas besitzt, und ein zweiter Widerstand eine Reaktion, selektiv oder in anderer Weise, einer anderen Art, n oder p, auf dasselbe Gas besitzt, und wobei die Widerstände derart miteinander verbunden sind, dass das Ausgangssignal die Differenz zwischen ihren momentanen Widerständen darstellt, **dadurch gekennzeichnet,** dass die Materialien des ersten und des zweiten Widerstandes aus folgenden Kombinationen ausgewählt sind:
(i) der erste und der zweite Widerstand sind aus SnO₂ bzw. Y₂TiFeO₇;
(ii) der erste und der zweite Widerstand sind aus SnO₂ bzw. Bi₂Sn₂O₇;
(iii) der erste und der zweite Widerstand sind aus BaSnO₃ bzw. Bi₂Sn₂O₇;
(iv) der erste und der zweite Widerstand sind aus FeNbO₄ bzw. Y₂TiFeO₇;
(v) der erste und der zweite Widerstand sind aus FeNbO₄ bzw. BiSn₂O₇;
(vi) die Vorrichtung ist für das selektive Erfassen von NO₂ geeignet, der erste Widerstand ist aus einem aus BaSnO₃, FeNbO₄ und SnO₂ ausgewählten Material und der zweite Widerstand ist aus einem aus Ba₆FeNb₆O₃₀ und Bi₆FeNb₉O₃₀ ausgewählten Material.

2. Verfahren zum Erfassen wenigstens eines Gases in einer Mischung mittels einer Anordnung von gaserfassenden Halbleiterwiderständen, dadurch gekennzeichnet, dass die Anordnung eine Anordnung gemäß Anspruch 1 ist und das Verfahren die Schritte des gleichzeitigen Zuführens der Mischung zum ersten Widerstand, um eine Reaktion der ersten Art zu erzeugen, und zum zweiten Widerstand, um eine Reaktion der anderen Art zu erzeugen, und des Messens der Differenz zwischen den resultierenden Widerstandssignalen von den Widerständen, aufweist.

3. Verfahren nach Anspruch 2, bei welchem die Materialien des ersten und des zweiten Sensors wie bei (vi) im Anspruch 1 definiert, ausgewählt sind, dadurch gekennzeichnet, dass die Differenz zwischen den resultierenden Widerstandssignalen als ein Maß für das Vorhandensein von NO₂ in der Mischung verwendet wird.

4. Verfahren nach Anspruch 2 oder 3, dadurch gekennzeichnet, dass die Differenz zwischen den Signalen gemessen wird, während die Widerstände auf verschiedenen Temperaturen sind.

## Revendications

1. Appareil pour détecter au moins un gaz dans un mélange, comprenant un réseau d'au moins deux résistances en semiconducteurs sensibles aux gaz, connectées pour donner un signal électrique de sortie à partir du réseau en réponse à la concentration d'au moins l'un desdits gaz, dans lequel une première résistance précitée a une réponse, sélective ou autre, d'un premier type, p ou n, à un gaz précité, et une seconde résistance précitée a une réponse, sélective ou autre, d'un autre type, n ou p, au même gaz, les résistances étant connectées de telle sorte que le signal de sortie représente la différence entre leurs résistances instantanées, caractérisé par le fait que les matières desdites première et seconde résistances sont choisies parmi les combinaisons suivantes :
(i) les première et seconde résistances sont respectivement de SnO₂ et Y₂TiFeO₇ ;
(ii) les première et seconde résistances sont respectivement de SnO₂ et Bi₂Sn₂O₇ ;
(iii) les première et seconde résistances sont respectivement de BaSnO₃ et Bi₂Sn₂O₇ ;
(iv) les première et seconde résistances sont respectivement de FeNbO₄ et Y₂TiFeO₇ ;
(v) les première et seconde résistances sont respectivement de FeNbO₄ et BiSn₂O₇ ;
(vi) l'appareil étant approprié pour la détection sélective de NO₂, la première résistance est faite d'une matière choisie parmi BaSnO₃, FeNbO₄ et SnO₂, et la seconde résistance est faite d'une matière choisie parmi Ba₆FeNb₉O₃₀ et Bi₆FeNb₉O₃₀.

2. Procédé de détection d'au moins un gaz dans un mélange au moyen d'un réseau de résistances en semiconducteurs sensibles aux gaz, caractérisé par le fait que le réseau est un réseau tel que défini à la revendication 1, le procédé comprenant les étapes consistant à introduire le mélange simultanément sur la première résistance pour produire une réponse dudit premier type et sur la seconde résistance pour produire une réponse dudit autre type, et à mesurer la différence entre les signaux de résistance résultants provenant des résistances.

3. Procédé selon la revendication 2, dans lequel les matières des premier et second détecteurs sont choisies comme défini au point (vi) à la revendication 1, caractérisé par le fait que la différence entre les signaux de résistance résultants est utilisée comme mesure de la présence de NO₂ dans le mélange.

4. Procédé selon la revendication 2 ou la revendication 3, caractérisé par le fait que la différence entre lesdits signaux est mesurée alors que les résistances sont à différentes températures.
